(19)

Europäisches Patentamt

European Patent Office

Office européen des brevets

(11) **EP 4 574 185 A1**

(12) **EUROPEAN PATENT APPLICATION**

(43) Date of publication:
   **25.06.2025   Bulletin 2025/26**

(21) Application number: **24220408.9**

(22) Date of filing: **17.12.2024**

(51) International Patent Classification (IPC):
   **A61M 1/34** (2006.01)          **A61M 1/36** (2006.01)
   **A61M 1/38** (2006.01)

(52) Cooperative Patent Classification (CPC):
   **A61M 1/3496; A61M 1/3672; A61M 1/3693;**
   **A61M 1/385;** A61M 2230/207

(84) Designated Contracting States:
   **AL AT BE BG CH CY CZ DE DK EE ES FI FR GB**
   **GR HR HU IE IS IT LI LT LU LV MC ME MK MT NL**
   **NO PL PT RO RS SE SI SK SM TR**
   Designated Extension States:
   **BA**
   Designated Validation States:
   **GE KH MA MD TN**

(30) Priority:   **18.12.2023   US 202363611759 P**

(71) Applicant: **Fenwal, Inc.**
   **Lake Zurich, IL 60047 (US)**

(72) Inventor: **PATEL, Amit J.**
   **Lake Zurich, 60047 (US)**

(74) Representative: **Maikowski & Ninnemann**
   **Patentanwälte Partnerschaft mbB**
   **Postfach 15 09 20**
   **10671 Berlin (DE)**

(54)   **SYSTEMS AND METHODS FOR CONSISTENT PLASMA ANTICOAGULANT PERCENTAGE**

(57)   A method for performing plasmapheresis using a blood separating system including a fluid flow circuit, a blood separator, and a controller. The method includes inputting a hematocrit value for a donor, setting a desired plasma anticoagulant percentage for a plasma product, calculating an initial ratio of anticoagulant to whole blood (ACR), and performing a separation with the separation system. A system is also provided comprising a programmable controller having a screen for receiving input from an operator and configured to provide, based on operator input, an initial ACR and operate the system at the initial ACR.

FIG. 1

## Description

## FIELD OF THE INVENTION

The present application relates to systems and methods for performing plasmapheresis and, more particularly, to plasmapheresis systems and methods wherein the plasma product includes a consistent plasma anticoagulant percentage.

## BACKGROUND

Plasmapheresis is an apheresis procedure in which whole blood is withdrawn from a donor, the plasma separated from the cellular blood components (red blood cells, platelets and leukocytes) and retained, and the cellular blood components returned to the donor. The separation of the plasma from the cellular components is typically accomplished in an automated procedure by centrifugation or membrane filtration.

In automated plasmapheresis, whole blood is drawn from the donor, mixed at a specified ratio with anticoagulant ("ACR"), and then separated into anticoagulated plasma and red blood cells and other cellular components.

Methods for determining the volume of raw plasma that may be withdrawn from any particular donor, consistent with donor safety and comfort, typically include an estimation/determination of the donor's total blood volume using donor-specific characteristics, and then determining the total volume of raw plasma that may be collected from that donor. See, e.g., WO 2019/226654 and USSN 17/078,824, filed October 23, 2020, both of which are incorporated by reference herein, and which disclose systems and methods for the optimization of plasma collection volumes based upon the use of donor-specific characteristics for determining a target collection volume of raw plasma for the donor.

The plasma anticoagulant percentage in the plasma product may vary based on donor characteristics and the mixing ratio of the whole blood and anticoagulant.

In plasmapheresis processing, plasmapheresis devices normally mix anticoagulant with drawn whole blood at a constant set ratio (ACR), such as 7:1 to 16:1. The anticoagulant mixes with the whole blood. As cellular content is separated, some anticoagulant remains with the plasma portion of the whole blood. The relative ratio of the anticoagulant to plasma is not constant, but dependent on the ratio of cellular content to plasma and varies based on donor hematocrit which may be dependent on the physical characteristics of the donor. Thus, the final anticoagulant plasma percentage in the plasma product may vary based on donor characteristics and the mixing ratio of the whole blood to anticoagulant (ACR).

The variation in the amount of anticoagulant in collected plasma may impact plasma quality due to dilution of the plasma at high anticoagulant percentages and possible clotting or flocculation at low anticoagulant percentages. Anticoagulant is also known to impact collected platelet quality during storage. Accordingly, reduction in the variability of anticoagulant in plasma the platelet storage medium should reduce variability in stored product quality.

It would, therefore, be desirable to achieve and ensure a consistent plasma anticoagulant percentage in the product. By way of the present application, a method and system are provided to achieve a consistent plasma anticoagulant percentage for the plasma product of a plasmapheresis procedure.

## SUMMARY

The present application has several aspects. In a first aspect, a method is provided for performing plasmapheresis using a blood separating system including a fluid flow circuit, a blood separator and a controller. The method includes inputting a first hematocrit value for a donor; setting a desired plasma anticoagulant percentage for a plasma product; calculating an initial ratio of anticoagulant to whole blood (ACR); and performing a separation with the separation system at the initial ACR. It is to be noted that the method may be conducted in an online modus (i.e. whole blood for plasmapheresis is withdrawn from any particular donor) or in an offline modus (i.e. whole blood is withdrawn from a container of previously collected blood of a donor). Offline processing might be also applicable to back-lab processing, such as separating red blood cells, platelet, and plasma from whole blood, and/or cell washing.

In a second aspect, an automated system for separating plasma from whole blood comprises a disposable fluid flow circuit including a separator for separating whole blood into a plasma product and a concentrated cell product and a reusable hardware component comprising a programmable controller having a display for receiving input from an operator and configured to provide, based on operator input, an initial ACR and operate the system at the initial ACR.

Other aspects will become apparent upon reference to the following description and accompanying drawings.

## BRIEF DESCRIPTION OF THE DRAWINGS

Fig. 1 is a perspective view of an exemplary plasmapheresis instrument suitable for use in the system and method of the present application.

Fig. 2 is a perspective view of a spinning membrane separator of the type incorporated in a disposable set, with portions broken away to show detail, usable with the plasmapheresis system of Fig. 1.

Fig. 3 is a perspective view of the front panel of the plasmapheresis system of Fig. 1 showing the components of the disposable set that are mounted thereto.

Fig. 4 is a schematic view showing operation of the plasmapheresis system in the collection phase.

Fig. 5 is a schematic view showing operation of the plasmapheresis system in the reinfusion phase.

Fig. 6 is a flow chart illustrating the steps of a method in accordance with the present application.

## DETAILED DESCRIPTION

A more detailed description of the systems and methods in accordance with the present disclosure is set forth below. It should be understood that the description below of a specific device and method is intended to be exemplary, and not exhaustive of all possible variations or applications. Thus, the scope of the disclosure is not intended to be limiting, and should be understood to encompass variations or embodiments that would occur to persons of ordinary skill.

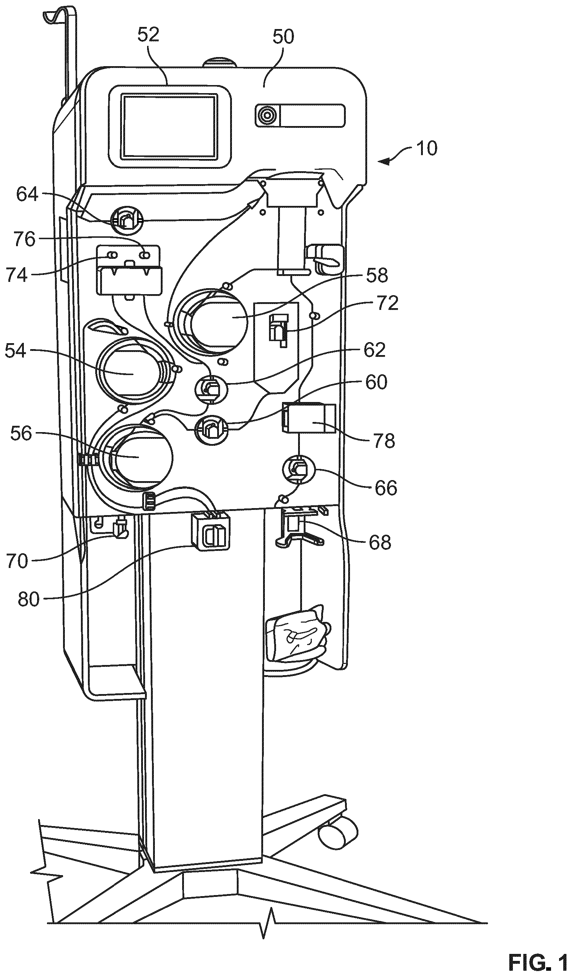

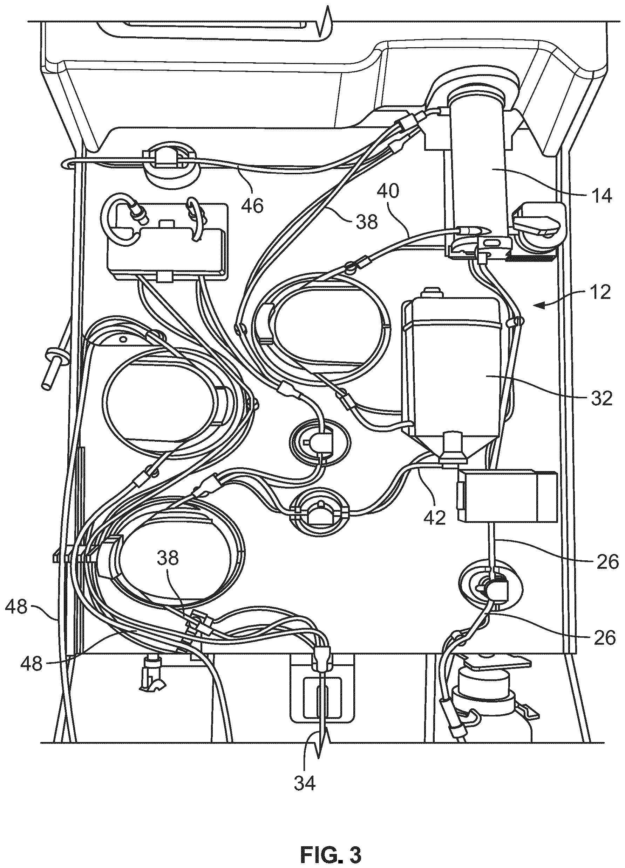

In the context of the present application, plasmapheresis is performed on an automated system comprising a hardware component, generally designated 10, and a disposable set, generally designated 12, to collect plasma. With reference to Figs. 1-5, and as described in greater detail below, the disposable set 12 consists of an integrally connected separator, containers, and tubing to transport blood and solutions within a sterile fluid pathway.

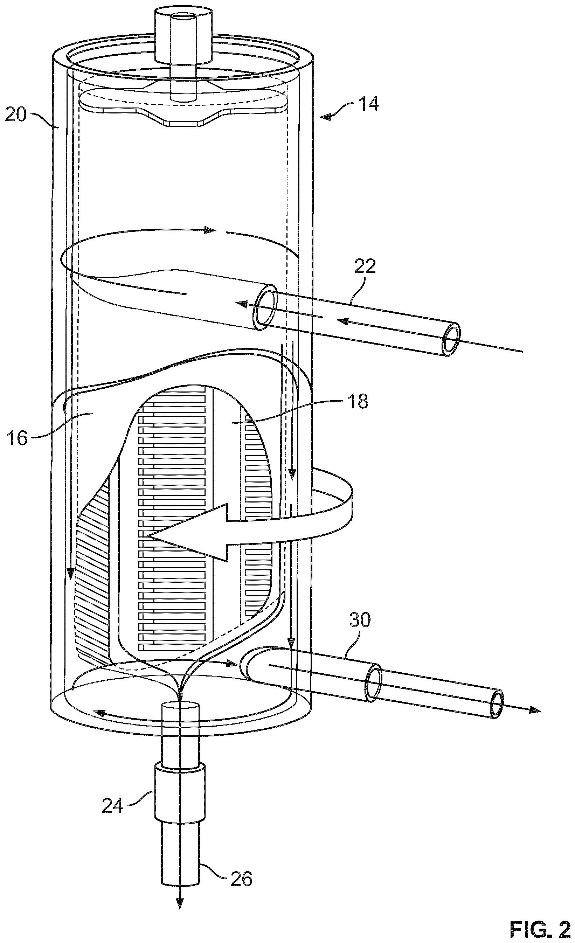

The separator 14, as seen in Fig. 2, includes a spinning membrane filter 16 mounted to a rotor 18 for rotation within a case 20 to separate blood into components. A detailed description of a spinning membrane separator may be found in US Pat. No. 5,194,145 to Schoendorfer, which is incorporated herein by reference. As can be appreciated, in a different system, separation of the whole blood may be accomplished by other separation means, such as centrifugation, as described for example in. US 5,360,542 to Williamson et al.

During plasmapheresis, whole blood is withdrawn from a donor, anticoagulant added, and the anticoagulated whole blood then enters the separator 14 through a whole blood input port 22. The plasma is separated by the spinning membrane filter, and then passes out of a plasma output port 24, through a plasma line 26, and into a plasma collection container 28. Concentrated cells are pumped out of a concentrated cell output port 30 into a reservoir 32, where the cells remain until reinfusion to the donor. As can be appreciated, some of the anticoagulant added to the whole blood will remain with the separated plasma, while another amount of anticoagulant will remain with the concentrated cells.

The disposable set 12 also includes tubing lines for introducing whole blood from the donor into the system during collection and returning concentrated cells to the donor during reinfusion (donor line 34, which terminates in the venipuncture needle 36), and for transporting anticoagulated whole blood to the separator (blood line 38), concentrated cells into the reservoir (cell line 40), concentrated cells from the reservoir to the donor line (reinfusion line 42), plasma into the plasma collection container (plasma line 44), saline (saline line 46), and anticoagulant (AC line 48).

The hardware component 10 includes a programmable controller 50 which is configured to control the operation of the system. The controller 50 may be provided as a computer or associated programmable microprocessor or other known mechanism for controlling one or more of the elements and processing information from one or more elements of the system in accordance with the procedure and steps set forth herein.

The controller 50 may be coupled to one or more of the structures of the hardware component 10, for example to receive information (e.g., in the form of signals) from these structures or to provide commands (e.g., in the form of signals) to these structures to control the operation of the structures. The controller 50 may be coupled to pumps, and the drive or separator to provide commands to those devices to control their operation. The controller 50 may be directly electrically connected to these structures to be coupled to them, or the controller 50 may be directly connected to other intermediate equipment that is directly connected to these structures to be coupled to them. The controller may also be wirelessly connected to any of these devices.

The controller may be connected to at least one input. The at least one input 52 may include a number of different devices according to the embodiments described herein. The input provides a mode of communication from the operator to the device and the device to the operator. For example, the input 52 could include a keyboard, keypad or touchscreen by which a user may provide information and/or instructions to the controller 50. If the input is a touchscreen, the touchscreen may be a graphical user interface ("GUI") through which the operator controls the procedure. For example, the GUI permits entry of any of a donor ID, donor sex, donor height, donor weight, donor age, donor hematocrit/hemoglobin; a target saline infusion volume (if a saline protocol is selected), a target plasma volume, and a target plasma anticoagulant percentage for the plasma product. The input 52 also enables the operator to gather status information and handle error conditions. The input 52 could also include a reader or scanner, such as a barcode reader or scanner or an RFID reader. The assembly of the input/touch screen may be one of the aforementioned structures to which the controller 50 is coupled from which the controller 50 receives information and to which the controller 50 provides commands. According to still other embodiments, the input 52 may be in the form of computer equipment that permits the blood draw system including the controller 50 to communicate (whether via wires, cables, etc. or wirelessly) with other systems over a local network, or with other cell processing systems or other computer equipment (e.g., a server) over local networks, wide area networks, or the Internet. According to such an embodiment, the input 52 may include an internal transmitter/receiver device.

Using the input 52, a phlebotomist or operator may enter parameters and information relevant to the plasmapheresis procedure. This information can include patient measurements such as height, weight, hematocrit, or characteristics such as gender, previous donation reactions, or any other relevant information. The information can also include desired product compositions, such as plasma anticoagulant product percentage. These measurements can be entered at the beginning of the procedure and also during the procedure. The controller may use these adjusts to calculate properties of the blood draw, such as an initial ACR value and a second or final ACR value. These set values can be calculated from the phlebotomist or operator input and patient characteristics. Hence, while it may be described herein that a particular component of the system performs a particular function, it should be understood that that component is being controlled by the controller to perform that function. The controller may also be connected to a patient monitoring device. Before, during, and after a procedure, the controller may receive readings from the patient monitoring device and process this information before displaying information regarding the patient or blood draw.

A plurality of peristaltic pumps (three are shown) are located on the front panel of the hardware component 10, including an AC pump 54, a blood pump 56, and a cell pump 58. The AC pump 54 delivers anticoagulant solution (AC) at a controlled rate into the blood line 38 as whole blood enters the set from the donor. The blood pump 56 delivers anticoagulated whole blood to the separator during the collection phase of the procedure and returns concentrated cellular components and, if desired, replacement fluid to the donor during the reinfusion phase of the procedure. The cell pump 58 delivers concentrated cellular components from the separator 14 to a reservoir during the collection phase.

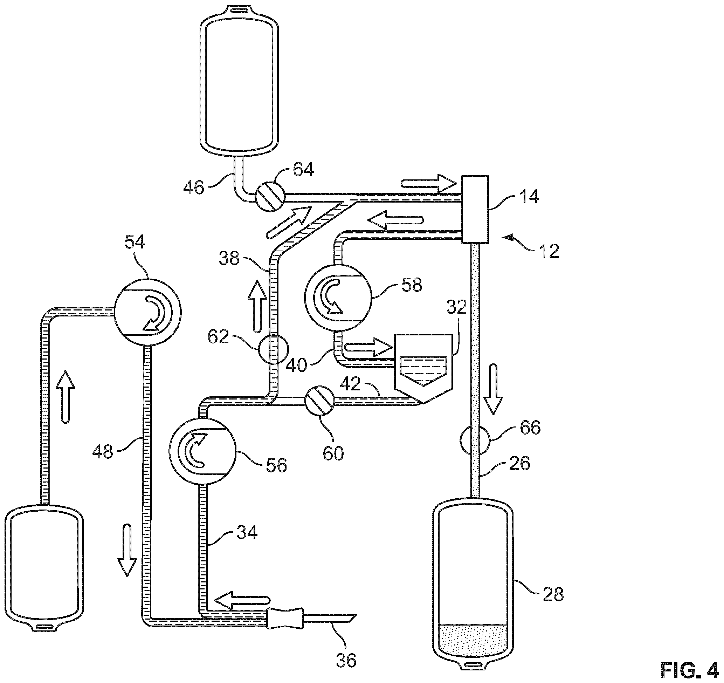

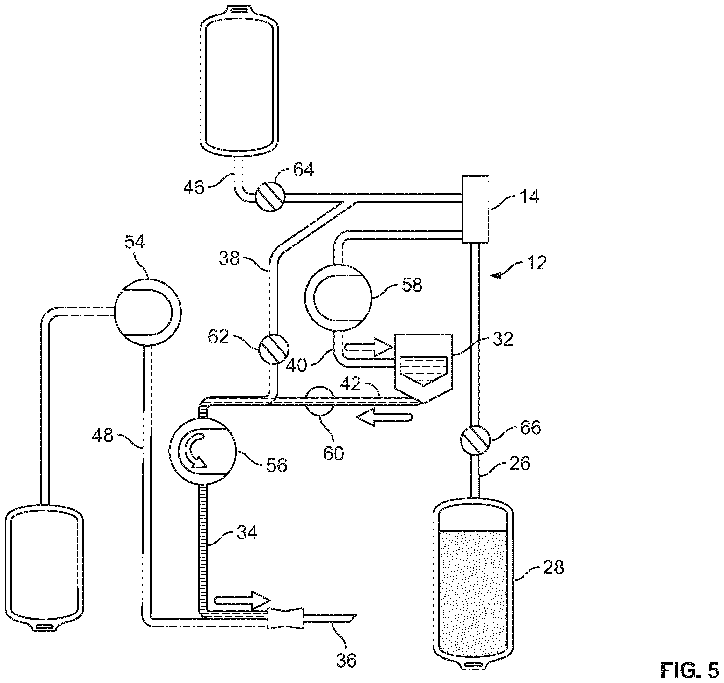

The front panel also includes multiple clamps (four are shown) into which the disposable set 12 is installed, including a reinfusion clamp 60, a blood clamp 62, a saline clamp 64, and a plasma clamp 66. The reinfusion clamp 60 closes to block the reinfusion line (42) during the collection phase (Fig. 4) and is open during the reinfusion phase (Fig. 5) to allow the blood pump to reinfuse the concentrated cellular components from the reservoir 32 to the donor. The blood clamp 62 opens during the collection phase to allow anticoagulated whole blood to be pumped to the separator 14 and closes during the reinfusion phase to block the blood line 38. The saline clamp 64 closes to block the saline line 46 during the collection phase and during reinfusion of the separated cellular components. If saline is to be used as a replacement fluid, the saline clamp 64 opens during the reinfusion phase. The plasma clamp 66 opens during the collection phase to allow plasma to flow into the plasma collection container 28 and closes during the reinfusion phase.

The hardware component 10 includes one or more (e.g., three, as shown) weigh scales to monitor the current plasma collection volume (scale 68), the AC solution volume (scale 70), and the concentrated cellular content volume (scale 72). The system also includes various sensors and detectors, including a venous pressure sensor 74, a separator pressure sensor 76, optical blood detectors 78, and an air detector 80.

The donor is connected to the system throughout the procedure. As illustrated, the disposable set 12 includes a single venipuncture needle 36, through which whole blood is drawn from the donor in a collection phase (Fig. 4) and concentrated cells are returned to the donor in a reinfusion stage (Fig. 5). As noted above, the plasmapheresis procedure may comprise a plurality of cycles each having a collection/separation phase followed by a return or reinfusion phase. During the collection phase, the whole blood is separated into plasma and concentrated cells. The disposable set includes a plasma collection container 28 for receipt of the separated plasma and a reservoir 32 for receipt of the concentrated cells. During the reinfusion phase, the concentrated cells from the reservoir 32 are reinfused to the donor through the venipuncture needle 36. Typically, plasmapheresis performed with a single venipuncture needle 36 involves multiple cycles of collection and reinfusion.

Returning to Fig. 4, during the collection phase, anticoagulant solution (AC) is pumped at a specified and set rate and mixed with whole blood as it enters the disposable set 12. The anticoagulated blood is pumped to the separator 14, where plasma is separated from the cellular components and directed to the plasma collection container 28.

The cellular components are pumped from the separator 14 to the reservoir 32. The collection phase stops when the reservoir 32 reaches an expected volume of concentrated cells or if the target plasma collection volume has been achieved.

Then, the reinfusion phase begins. With reference to Fig. 5, during the reinfusion phase, the blood pump 56 reverses direction and pumps the concentrated cells from the reservoir 32 back to the donor through the apheresis needle 36. If a saline protocol was selected, by which saline is returned to the donor as a replacement fluid for the collected plasma, the final reinfusion phase is followed by saline infusion.

Before beginning any plasmapheresis procedure, donor-specific characteristics may be determined and/or entered into the system to determine at least the appropriate initial anticoagulant/whole blood ratio (ACR) needed to achieve a desired plasma anticoagulant percentage. Plasma anticoagulant percentage is the percentage of anticoagulant in the total plasma/anticoagulant product. Plasma anticoagulant percentage is a volume percentage or measure of the concentration of a substance in a solution. The desired plasma anticoagulant percentage is set by the user and may be a set range or value. In one embodiment, the plasma anticoagulant percentage is from about 7-14%. More particularly, the desired plasma anticoagulant percentage is between 9-12%. The plasma anticoagulant percentage can be set at any number between this range, such as 9.75, 10.1, etc.

The donor-specific characteristics used to arrive at anticoagulant/whole blood ratio (ACR) comprise at least the donor's hematocrit, as the hematocrit is needed to determine the percentage of anticoagulant that is collected along with the plasma in the plasma product container. Hematocrit (HCT) can also be used to calculate the appropriate ACR, based on a desired plasma anticoagulant percentage. The percentage of anticoagulant in the plasma/anticoagulant product (divided by 100) is equal to $1/(1 + ACR*(1- HCT/100))$. For example, if a 16% plasma anticoagulant percentage is preferred and the donor has a hematocrit of 38, the calculation is as follows:

$$.16= 1/(1+ACR(1-.38))$$

$$ACR= 8.5$$

However, if the donor's hematocrit is 55 and a 16% plasma anticoagulant percentage is still preferred, the calculation is as follows:

$$.16= 1/(1+ACR(1-.55))$$

$$ACR= 11.7$$

The hematocrit of the donor may be determined using any number of well-known methods, such as by use of a dedicated hematocrit centrifuge (e.g., the SciLogex DM4124 Hematocrit Centrifuge) or hematology analyzer (e.g., the Sysmex XP-300™ Automated Hematology Analyzer). Once determined, the initial ACR can be calculated and set for the procedure.

The donor hematocrit can also vary during the plasmapheresis procedure as a function of the amount of plasma collected. More specifically, because the amount of donor plasma is depleted as the procedure progresses, the donor's hematocrit will be higher during the later part of the procedure. Accordingly, in order to maintain a consistent plasma percentage in the product, the ACR will need to be adjusted during the procedure. Thus, the ACR based on the donor's initial hematocrit may be increased to account for the increasing hematocrit. In order to estimate this change during the procedure, donor characteristics, such as height and weight can be used to provide an estimate of the change. The change in hematocrit can be estimated by calculating the donor's total blood volume and by monitoring the volume of plasma collected.

A donor's total blood volume may be determined using the donor's weight and height, by using, for example the Lemmens formula, in which the total blood volume is based on the BMI (Body Mass Index)

$$TBV = 70/\sqrt{\left(\frac{BMI}{22}\right)}$$

of the donor ( ).

In a further alternative, a donor's total blood volume may be determined using the donor's weight, height, and gender, buy using, for example, the Nadler equations, with the donor's hematocrit then being applied as described above to determine the total plasma volume of the donor and the target volume of plasma to be collected.

As can be appreciated, the methodologies for determining total blood volume described above are exemplary. Any other generally accepted methodology for determining donor's total blood volume may also be used, such as any of those described in US 2020/0147289, which is incorporated herein by reference.

If all of the donor's required measurements for calculating total blood volume are not available, an estimation method may be used. For instance, when the donor's weight and a hematocrit value are the only donor-specific characteristics used, a three-tier nomogram, such as the FDA nomogram described above, can be adopted, in which three different weight classes of donors are utilized (between 110 lbs. and 149 lbs., between 150 lbs. and 174 lbs., and 175 lbs. and up).

The total blood volume may then be used to determine a second or final hematocrit for a donor. By way of example, if the donor's total blood volume is determined to be 5000 mL, and the donor's hematocrit at the beginning of the procedure is 40%, red blood cells will constitute 2000 mL and plasma 3000 mL. After 500 mL of plasma has been collected and the associated red blood cells reinfused to the donor, the donor will have a total blood volume of 4500 mL, with 2000 mL of red blood cells and 2500 mL of plasma, for a hematocrit of 44.4% (2000 mL/(2000mL + 2500 mL), or an increase in hematocrit of 4.4%. As a further example, for the same donor with a total blood volume of 5000 mL, if 500 mL of plasma has been collected and the associated red blood cells reinfused to the donor along with 50 mL of anticoagulant, the donor will have a total blood volume of 4550 mL, with 2000 mL of red blood cells for a hematocrit of 43.9% (2000mL/4550mL), or an increase of 3.9%. The increase in hematocrit will be a function of the size of the donor (for larger donors having a larger total blood volume and, consequently, a larger total plasma volume, the change in hematocrit will be less), and how much plasma has been collected. Due to the increase in hematocrit, the appropriate ACR will need to be adjusted. Thus, based on the targeted plasma collection volume, a "final' or second hematocrit of the donor can be estimated, which will be higher than the initial hematocrit. These values can be used to generate an initial ACR and a second or final ACR. Alternatively, a hematocrit for the entire procedure may be based on an average between the initial and final hematocrits of the donor, and the average value used when determining the ACR (average ACR) for the procedure.

Alternatively, hematocrit may be measured before the beginning of the procedure for an initial hematocrit and at a later point during the procedure for a final or second hematocrit. The later point may be halfway through the procedure or another designated time. This time may be based on the volume of the plasma product (provided by the scale 68 for the plasma product), or when a certain volume of plasma has been collected. If the second hematocrit is measured instead of approximated, the second hematocrit will need to be entered at the input 52. The controller may prompt a user to enter the second hematocrit at a noted time in the procedure, such as the halfway point or when the plasma product container reaches a certain volume.

The donor-specific characteristics may be entered into the controller by the operator using the input 52. Alternatively, the donor-specific characteristics may be provided to the controller through a data management system that includes a database including such information. If the hematocrit is measured during the procedure, the controller may be programmed to prompt a user to enter a second hematocrit value.

As can be appreciated, many of the steps that are performed in arriving at a predicted initial ACR, second ACR, or average ACR, may be pre-programmed into the controller of the system so that they can be performed automatically upon appropriate input by the operator of the relevant donor-specific characteristics and/or feedback from the system, such as volume or weight of the plasma product container.

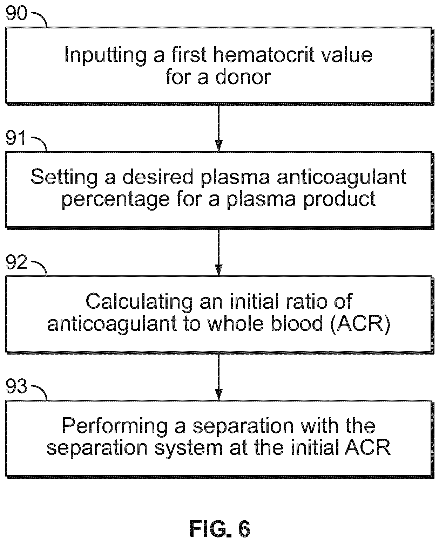

The steps of the method disclosed herein are illustrated by way of the flow chart comprising Fig. 6 of this application. With reference to Fig. 6, the method comprises a first step of inputting a hematocrit for a donor (Box 90); a second step of setting a desired plasma anticoagulant percentage for the product (Box 91); a third step of calculating an initial anticoagulant to whole blood ratio (ACR) (Box 92), and a fourth step of performing a separation with the separation system at the first ACR (Box 93). In accordance with the method, each of steps illustrated in Boxes 90-92 being performed prior to connecting the donor to the fluid flow circuit. As can be appreciated once the hematocrit is measured and entered into the controller and the desired plasma anticoagulant percentage is entered, either by the operator or a data management system, the controller can automatically perform and make a recommendation as to ACR that is used for the procedure, with a first and second ACR possibly being utilized. The second ACR can be estimated by the controller based on total blood volume from known donor characteristics and monitoring product volume or it can be calculated based on a second measured hematocrit of the donor.

It will be understood that the embodiments described are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope of the claims is not limited to the above-description, but is set forth in the following claims.

Aspects

Aspect 1. A method for performing plasmapheresis using a blood separating system including a fluid flow circuit, a blood separator and a controller, comprising: inputting a first hematocrit value for a donor; setting a desired plasma anticoagulant percentage for a plasma product; calculating an initial ratio of anticoagulant to whole blood (ACR); and performing a separation with the separation system at the initial ACR.

Aspect 2. The method of Aspect 1, wherein the first hematocrit of the donor is measured prior to inputting the value.

Aspect 3. The method of Aspect 1, wherein calculating an initial ratio of anticoagulant to whole blood is done using the formula: $(PA\%/100) = 1/(1 + ACR*(1-Hct/100))$; wherein PA% is the plasma anticoagulant percentage and ACR is the ratio of anticoagulant to whole blood and Hct is the hematocrit of the donor.

Aspect 4. The method of Aspect 1, wherein the plasma anticoagulant percentage is between 7% and 14%.

Aspect 5. The method of Aspect 4, wherein the plasma anticoagulant percentage is between 9% and 12%.

Aspect 6. The method of any of the preceding Aspects, wherein steps a-c are done before connecting a donor to a system.

Aspect 7. The method of any of the preceding Aspects, further comprising imputing at least one additional donor characteristic with the hematocrit.

Aspect 8. The method of Aspect 7, wherein the at least one additional donor characteristic includes weight of the donor.

Aspect 9. The method of Aspect 7 or Aspect 8, wherein the at least one additional donor characteristic includes the height of the donor.

Aspect 10. The method of Aspect 7, wherein the at least one donor characteristic is used to calculate a second ACR and the controller changes the ACR from the initial ACR to the second ACR at a set point in the separation procedure.

Aspect 11. The method of Aspect 10, wherein the set point is about half way through the procedure.

Aspect 12. The method of Aspect 10, wherein the set point is at a designated plasma product volume.

Aspect 13. The method of Aspect 10, wherein the equation used to calculate the second ACR includes calculating a total blood volume from the height and weight of the donor and a predicted change in the hematocrit for the donor.

Aspect 14. An automated system for separating plasma from whole blood comprising a disposable fluid flow circuit including a separator for separating whole blood into a plasma fraction and a concentrated cell fraction and a reusable hardware component comprising a programmable controller having a display for receiving input from an operator and configured to provide, based on operator input, an initial ACR and operate the system at the initial ACR.

Aspect 15. The automated system of Aspect 14, wherein the programmable controller is configured to calculate a second ACR and operate the system at the second ACR at a set point in a separation procedure.

Aspect 16. The automated system of Aspect 15, wherein the set point is halfway through the separation procedure.

Aspect 17. The automated system of Aspect 15, wherein the set point is at a designated plasma product volume.

Aspect 18. The automated system of Aspect 15, wherein the programmable controller calculates a second ACR based on at least one donor characteristic.

Aspect 19. The automated system of Aspect 18, wherein the at least one donor characteristic includes weight of the donor.

Aspect 20. The automated system of Aspect 18 or Aspect 19, wherein the at least one donor characteristic includes the height of the donor.

**Claims**

1. An automated system for separating plasma from whole blood comprising a disposable fluid flow circuit including a separator for separating whole blood into a plasma product and a concentrated cell fraction and a reusable hardware component comprising a programmable controller having an input for receiving information from an operator and configured to provide, based on operator input, an initial ratio of anticoagulant to whole blood (ACR) and operate the system at the initial ACR.

2. The automated system of claim 1, wherein the initial ACR is calculated using a hematocrit of the donor of the whole blood.

3. The automated system of claim 1, wherein the initial ACR is calculated using the formula: (PA %/100) = 1/(1 + ACR*(1-Hct/100)); wherein PA% is the plasma anticoagulant percentage and ACR is the ratio of anticoagulant to whole blood and Hct is the hematocrit of the donor of the whole blood.

4. The automated system of claim 3, wherein the plasma anticoagulant percentage is between 7% and 14%.

5. The automated system of claim 4, wherein the plasma anticoagulant percentage is between 9% and 12%.

6. The automated system of claim 1, wherein the programmable controller is configured to calculate a second ACR and operate the system at the second ACR at a set point in separating the whole blood into a plasma product.

7. The automated system of claim 6, wherein the set point is halfway through the separating of the whole blood into a plasma product.

8. The automated system of claim 6, wherein the set point is at a designated plasma product volume.

9. The automated system of claim 6, wherein the programmable controller calculates a second ACR based on at least one donor characteristic of the donor of the whole blood.

10. The automated system of claim 9, wherein the at least one donor characteristic includes weight of the donor of the whole blood.

11. The automated system of claim 9 or claim 10, wherein the at least one donor characteristic includes the height of the donor of the whole blood.

12. The automated system of claim 9, wherein the at least one donor characteristic includes the hematocrit of the donor of the whole blood.

13. The automated system of claim 9, wherein the at least one donor characteristic is provided to the controller through a data management system that includes a database including such information.

14. The automated system of claim 9, wherein the at least one donor characteristic is entered by the operator.

15. The automated system of claim 1, wherein the separator is a spinning membrane separator.

FIG. 1

FIG. 2

**FIG. 3**

FIG. 4

FIG. 5

90 ⌐

Inputting a first hematocrit value
for a donor

91 ⌐

Setting a desired plasma anticoagulant
percentage for a plasma product

92 ⌐

Calculating an initial ratio of
anticoagulant to whole blood (ACR)

93 ⌐

Performing a separation with the
separation system at the initial ACR

**FIG. 6**

Europäisches
Patentamt

European
Patent Office

Office européen
des brevets

# EUROPEAN SEARCH REPORT

**Application Number**

EP 24 22 0408

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/226654 A1 (FENWAL INC [US]) 28 November 2019 (2019-11-28) | 1,2,15 | INV.<br>A61M1/34 |
| Y | * claims 1,18,3 * | 3-5 | A61M1/36 |
| A | | 6-14 | A61M1/38 |
| | ----- | | |
| Y | WO 2022/159584 A1 (FENWAL INC [US]) 28 July 2022 (2022-07-28) * paragraph [0051] * | 3-5 | |
| | ----- | | |
| A | WO 2019/084278 A1 (HAEMONETICS CORP [US]) 2 May 2019 (2019-05-02) * paragraph [0071] * | 1-15 | |
| | ----- | | |

**TECHNICAL FIELDS SEARCHED (IPC)**

A61M

The present search report has been drawn up for all claims

| Place of search | Date of completion of the search | Examiner |
|---|---|---|
| The Hague | 21 March 2025 | Vadot, Pierre |

EPO FORM 1503 03.82 (P04C01)

## ANNEX TO THE EUROPEAN SEARCH REPORT
## ON EUROPEAN PATENT APPLICATION NO.

EP 24 22 0408

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2025

| Patent document cited in search report | Publication date | Patent family member(s) | Publication date |
|---|---|---|---|
| WO 2019226654 A1 | 28-11-2019 | AU 2019274489 A1 | 26-11-2020 |
| | | AU 2020267188 A1 | 03-12-2020 |
| | | AU 2021204735 A1 | 05-08-2021 |
| | | AU 2023270336 A1 | 14-12-2023 |
| | | CA 3099428 A1 | 28-11-2019 |
| | | CN 112105403 A | 18-12-2020 |
| | | CN 112587740 A | 02-04-2021 |
| | | CN 115300694 A | 08-11-2022 |
| | | DK 3621674 T3 | 06-12-2021 |
| | | EP 3621674 A1 | 18-03-2020 |
| | | EP 3884972 A1 | 29-09-2021 |
| | | EP 3925640 A1 | 22-12-2021 |
| | | EP 3954407 A1 | 16-02-2022 |
| | | EP 4186540 A1 | 31-05-2023 |
| | | ES 2900207 T3 | 16-03-2022 |
| | | ES 2951551 T3 | 23-10-2023 |
| | | HU E056564 T2 | 28-02-2022 |
| | | HU E062925 T2 | 28-12-2023 |
| | | JP 6976458 B2 | 08-12-2021 |
| | | JP 7098702 B2 | 11-07-2022 |
| | | JP 7520915 B2 | 23-07-2024 |
| | | JP 2021094384 A | 24-06-2021 |
| | | JP 2021515679 A | 24-06-2021 |
| | | JP 2022130592 A | 06-09-2022 |
| | | KR 20200136041 A | 04-12-2020 |
| | | KR 20200136488 A | 07-12-2020 |
| | | KR 20220000947 A | 04-01-2022 |
| | | KR 20220132645 A | 30-09-2022 |
| | | PL 3621674 T3 | 31-01-2022 |
| | | RU 2752596 C1 | 29-07-2021 |
| | | SA 520420610 B1 | 29-01-2024 |
| | | US 2020147289 A1 | 14-05-2020 |
| | | US 2021008272 A1 | 14-01-2021 |
| | | US 2021015989 A1 | 21-01-2021 |
| | | US 2021187182 A1 | 24-06-2021 |
| | | US 2021353841 A1 | 18-11-2021 |
| | | US 2022168486 A1 | 02-06-2022 |
| | | US 2022280708 A1 | 08-09-2022 |
| | | US 2023181807 A1 | 15-06-2023 |
| | | US 2024374804 A1 | 14-11-2024 |
| | | WO 2019226654 A1 | 28-11-2019 |
| WO 2022159584 A1 | 28-07-2022 | EP 4281136 A1 | 29-11-2023 |
| | | JP 2024504381 A | 31-01-2024 |
| | | US 2024082471 A1 | 14-03-2024 |
| | | WO 2022159584 A1 | 28-07-2022 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**                    EP 24 22 0408

This annex lists the patent family members relating to the patent documents cited in  the above-mentioned European search report.
The members are as contained in the European Patent Office EDP file on
The European Patent Office is in no way liable for these particulars which are merely given for the purpose of information.

21-03-2025

| Patent document cited in search report | Publication date | Patent family member(s) | Publication date |
|---|---|---|---|
| WO 2019084278 A1 | 02-05-2019 | AU 2018355417 A1 | 14-05-2020 |
| | | AU 2025200662 A1 | 20-02-2025 |
| | | CA 3079851 A1 | 02-05-2019 |
| | | CN 111465421 A | 28-07-2020 |
| | | CN 117959513 A | 03-05-2024 |
| | | EP 3700606 A1 | 02-09-2020 |
| | | JP 7390287 B2 | 01-12-2023 |
| | | JP 2021500950 A | 14-01-2021 |
| | | JP 2023154074 A | 18-10-2023 |
| | | KR 20200078553 A | 01-07-2020 |
| | | KR 20240017096 A | 06-02-2024 |
| | | SA 520411828 B1 | 05-03-2024 |
| | | WO 2019084278 A1 | 02-05-2019 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019226654 A
- US 07882420
- US 5194145 A, Schoendorfer
- US 5360542 A, Williamson
- US 20200147289 A